# EUROPÄISCHE PATENTSCHRIFT

(11) **EP 2 699 211 B1**
(45) Veröffentlichungstag und Bekanntmachung des Hinweises auf die Patenterteilung: **02.03.2022**
(21) Anmeldenummer: 12713681.0
(22) Anmeldetag: 30.03.2012
(51) Int. Cl.: A61F 13/15, A61F 13/505

(54) **FIXIER- UND TRAGEVORRICHTUNG FÜR EINE WEGWERFBARE ABSORBIERENDE INKONTINENZVORLAGE MIT GUTEM PRODUKTSITZ**
FASTENING AND CARRYING DEVICE FOR A DISPOSABLE ABSORBENT INCONTINENCE PAD HAVING GOOD FIT
DISPOSITIF DE FIXATION ET DE SUPPORT POUR UNE PROTECTION ABSORBANTE JETABLE POUR INCONTINENCE PRÉSENTANT UN BON CONFORT

(30) Priorität: 20.04.2011 DE 102011007818
(43) Veröffentlichungstag der Anmeldung: 26.02.2014
(73) Patentinhaber: Paul Hartmann AG, 89522 Heidenheim (DE)
(72) Erfinder: PAZ, Rui Miguel, 47800 Krefeld (DE); KESSELMEIER, Ruediger, 89233 Burlafingen (DE); MALOWANIEC, D. Krzysztof, 89522 Heidenheim (DE); DRUMEVA-EBERIUS, Albena, 89129 Langenau (DE); NAGY, Uwe, 89522 Heidenheim (DE); SWEREV, Maximilian, 86169 Augsburg (DE)
(74) Vertreter: DREISS Patentanwälte PartG mbB
(86) Internationale Anmeldenummer: PCT/EP2012/055872
(87) Internationale Veröffentlichungsnummer: WO 2012/143227

(56) Entgegenhaltungen:
- CH-A- 278 262
- US-A- 4 022 212
- US-A1- 2006 247 599

## Beschreibung

Die Erfindung betrifft eine Fixier- und Tragevorrichtung für eine wegwerfbare absorbierende Inkontinenzvorlage, mit einem mittels Gürtelverschlusselementen auf sich selbst lösbar schließbaren und so eine in Hüftumfangsrichtung durchgehende Hüftöffnung bildenden Hüftgürtel, an den die Inkontinenzvorlage lösbar fixierbar ist, so dass sie im Schrittbereich des Benutzers getragen und nach Gebrauch wieder vom Hüftgürtel entfernt und verworfen werden kann, wobei der Hüftgürtel einen vorderen Bauchbereich, einen hinteren Rückenbereich und einen linken und einen rechten Seitenbereich umfasst, wobei der Hüftgürtel ausgehend von dem Rückenbereich und dem Bauchbereich je einen vorzugsweise symmetrisch zu einer Längsmittelachse vorgesehenen und sich in einer Längsrichtung in Richtung auf den Schrittbereich des Benutzers erstreckenden Latzabschnitt aufweist, der an seiner körperzugewandten Seite vorzugsweise mechanisch und/oder klebend wirkende Verschlusselemente aufweist, die mit komplementären vorzugsweise mechanisch und/oder klebend wirkenden Verschlusselementen auf der körperabgewandten Seite der Inkontinenzvorlage lösbar haftend zusammenwirken, wobei die Inkontinenzvorlage einen Rückenbereich und einen Bauchbereich aufweist, deren Abmessungen durch die Breite der Inkontinenzvorlage quer zur Längsrichtung sowie die Erstreckung in Längsrichtung von einer Querkante der Inkontinenzvorlage bis zu einer distalen Kante des die Inkontinenzvorlage überdeckenden vorderen oder hinteren Latzabschnitts bestimmt ist.

Eine derartige Fixier- und Tragevorrichtung ist beispielsweise bekannt aus WO 2004/069122 A1. Bei dieser bekannten Fixier- und Tragevorrichtung wird der Hüftgürtel im Bauchbereich auf sich selbst geschlossen. In Seitenbereichen links und rechts zwischen Bauchbereich und Rückenbereich erstreckt sich der Hüftgürtel nach unten und bildet dort jeweils einen latzartigen Ansatz, an den eine verhältnismäßig breit ausladende absorbierende Windeleinheit festgelegt werden kann, wobei auf der körperzugewandten Seite der absorbierenden Windeleinheit beidseits vorn und hinten Verschlusselemente hierfür vorgesehen sind, festgelegt werden.

Aus EP 0 700 278 B1 ist eine sogenannte Gürtelwindel mit einem auf sich selbst schließbaren verhältnismäßig schmalen Hüftgürtel bekannt, an dessen körperabgewandter Seite eine nach Art einer Windel anmutende absorbierende Einheit festlegbar ist.

Eine gattungsgemäße Vorrichtung zeigt z.B. die DE 10 2009 049 463. US 2006/247599 A1 offenbart eine Fixer- und Tragevorrichtung für eine wegwerfbare absorbierende Inkontinenzvorlage.

Der vorliegenden Erfindung liegt die Aufgabe zugrunde, eine Fixier- und Tragevorrichtung der eingangs genannten Art zu schaffen, die im angelegten Zustand der Inkontinenzvorlage einen guten Produktsitz bereitstellt.

Diese Aufgabe wird ausgehend von einer Fixier- und Tragevorrichtung sowie im unabhängigen Anspruch 1 definiert gelöst.

Durch den in Richtung auf den Schrittbereich des Benutzers erstreckten Latzabschnitt bildet der Hüftgürtel zugleich die grundsätzliche Konfiguration eines Höschens, welches zur Vollendung der Höschenform nur noch durch einen Schrittbereich ergänzt zu werden braucht. Dieser Schrittbereich wird bei der erfindungsgemäßen Fixier- und Tragevorrichtung durch die absorbierende Inkontinenzvorlage gebildet, wobei der betreffende hintere und vordere Latzabschnitt mit der körperabgewandten Seite der Inkontinenzvorlage lösbar haftend zusammenwirkt, und zwar unter Verwendung von vorzugsweise mechanisch und/oder klebend wirkenden Verschlusselementen.

Durch die Überdeckung des Rückenbereichs der Inkontinenzvorlage durch den hinteren Rückenbereich des Hüftgürtels und den hieran anschließenden Latzabschnitt um wenigstens 50% wird erreicht, dass eine optimale Abdeckung der Inkontinenzvorlage in deren Rückenbereich erreicht wird, wodurch der Produktsitz optimiert und so ein Verrutschen der Inkontinenzvorlage verhindert oder reduziert werden kann. Auf diese Weise wird zugleich die Gefahr des Auslaufens minimiert.

Besonders bevorzugt wird der Rückenbereich der Inkontinenzvorlage durch den hinteren Rückenbereich des Hüftgürtels und/oder den hinteren Latzabschnitt zu wenigstens 60% und besonders bevorzugt zu wenigstens 70% überdeckt. Durch diese noch größere Überdeckungsrate werden die vorstehend genannten Vorteile weiter verstärkt.

Weiterhin ist es bevorzugt, wenn auch im Bauchbereich der Inkontinenzvorlage diese durch den Bauchbereich des Hüftgürtels und/oder den mit dem Bauchbereich des Hüftgürtels verbundenen Latzabschnitt um mindestens 50%, vorzugsweise mindestens 60% und insbesondere mindestens 70% überdeckt ist.

Als Längsrichtung des Hüftgürtels wird dabei die Längsrichtung an einem stehenden Benutzer definiert, die Querrichtung ist dabei senkrecht hierzu und fällt mit der Hüftumfangsrichtung zusammen. Die Längsrichtung der Inkontinenzvorlage entspricht der Richtung ihrer Längserstreckung, wobei die Querrichtung hierzu senkrecht verläuft.

Besonders bevorzugt weist die Inkontinenzvorlage zwei Längs- und zwei Querkanten auf, wobei im an der Fixier- und Tragevorrichtung festgelegten Zustand die Querkanten der Inkontinenzvorlage vorzugsweise im Wesentlichen einer Querkante, insbesondere der dem Schrittbereich abgewandten, also proximalen Querkante des Hüftgürtels entsprechen. Das heißt, die proximale Kante des Bauch- oder Rückenbereichs fällt vorzugsweise mit einer Querkante der Inkontinenzvorlage in angelegten Zustand zusammen. Im Falle einer konturierten proximalen Querkante des Hüftgürtels, beispielsweise durch einen Bauchausschnitt, läuft die proximale Kante parallel zur Hüftumfangsrichtung durch den distalsten Punkt dieser Konturierung. Auf diese Weise kann einfach eine Markierung bereitgestellt werden, anhand derer die optimale Anlegung des Hüftgürtels sowie der Inkontinenzvorlage am Hüftgürtel erreicht werden kann und somit auch für die jeweilige Größe optimale Positionierung der Verschlusselemente, um einen auf der einen Seite optimalen Aufspanneffekt der Materialabschnitte und damit der dem Träger zugewandten Lage zu erreichen und auf der anderen Seite einen Kontakt der Verschlusselemente mit dem Benutzer zu verhindern.

Vorzugsweise sind die Verschlusselemente in dieser Position vorzugsweise 100 mm bis 180 mm, vorzugsweise 120 mm bis 170 mm, weiter bevorzugt 140 mm bis 160 mm im Bereich des vorderen Latzabschnitts und vorzugsweise 170 mm bis 250 mm, vorzugsweise 190 mm bis 240 mm, weiter bevorzugt 210 mm bis 230 mm im Bereich des hinteren Latzabschnitts von der proximalen Querkante des Hüftgürtels entfernt.

Die vorstehend genannten Abdeckungsraten werden jedoch vorzugsweise auch erreicht, wenn die Querkanten der Inkontinenzvorlage nicht bündig mit den Querkanten des Hüftgürtels, insbesondere im Rückenbereich übereinander liegen, sondern die Querkante der Inkontinenzvorlage in proximaler Richtung über die Querkante des Hüftgürtels, insbesondere im Rückenbereich hinausragt oder die Querkante des Hüftgürtels in proximaler Richtung über die Querkante der Inkontinenzvorlage, insbesondere im Rückenbereich hinausragt.

Weiterhin umfasst die Inkontinenzvorlage eine Rückenschicht (Backsheet oder Außenseite), die vorzugsweise flüssigkeitsundurchlässig ist und eine körperzugewandte Schicht (Topsheet), die vorzugsweise flüssigkeitsdurchlässig ist sowie einen zwischen diesen beiden Schichten eingeschlossenen absorbierenden Kern, der eine äußeren Rand als Begrenzung aufweist.

Die Breite der Inkontinenzvorlage an der breitesten Stelle des Produktes beträgt von 200 mm bis 400 mm, vorzugsweise 220 mm bis 320 mm, die Breite des absorbierenden Kerns beträgt von 180 mm bis 380 mm, vorzugsweise 200 mm bis 300 mm. So beträgt beispielsweise die Breite der Inkontinenzvorlage MoliForm^{®} Premium Soft Light (Größe 1) 260 mm, die Breite des absorbierenden Kerns 242 mm, bei MoliForm^{®} Premium Soft Extra (Größe 3) beträgt die Breite der Vorlage 300 mm, die Breite des absorbierenden Kerns 260 mm.

Der Hüftgürtel umfasst in Hüftumfangsrichtung einen Bauchbereich sowie einen Rückenbereich und zwei Seitenbereiche, die jeweils eine Erstreckung in Hüftumfangsrichtung aufweisen und sich in Hüftumfangsrichtung aneinander anschließen bei Bildung eines geschlossenen Hüftgürtels.

In Längsrichtung besitzen Bauch- und Rückenbereich sowie die Seitenbereiche eine proximale und eine distale Querkante, die gerade oder gekrümmt, insbesondere auch nur bereichsweise gekrümmt ausgebildet sein können, zur Ausbildung einer Konturierung.

Schließlich wird die distale Erstreckung des Bauch- und Rückenbereichs und damit die distale Querkante des Bauch- und Rückenbereichs definiert durch eine gedachte Linie in Querrichtung am proximalsten Punkt der distalen Begrenzung des Hüftgürtels, beispielsweise eine Tangente. Der proximalste Punkt kann dabei in Hüftumfangsrichtung im Bauch-, Rücken- oder Seitenbereich liegen. Dabei ist die Fläche proximal dieser distalen Querkante ein Teil des Bauch- bzw. Rückenbereichs und die Fläche distal dieser distalen Querkante ein Teil des jeweiligen Latzabschnitts.

Als Längsrichtung des Hüftgürtels wird dabei die Längsrichtung an einem stehenden Benutzer definiert, die Querrichtung ist dabei senkrecht hierzu und fällt mit der Hüftumfangsrichtung zusammen. Die Längsrichtung der Inkontinenzvorlage entspricht der Richtung deren Längserstreckung, wobei die Querrichtung hierzu senkrecht verläuft.

Zur Verbesserung der Passform und für eine einfache Handhabung erweist es sich als vorteilhaft, wenn der betreffende Latzabschnitt sich in Richtung auf den Schrittbereich verjüngt, wobei die aufeinander zu laufenden Kanten der Latzabschnitte gerade oder gekrümmt sein können zur Ausbildung einer Konturierung. Durch die Konturierung, die insbesondere durch eine bogenförmige Krümmung der betreffenden Kanten, beispielsweise eine konkave Krümmung der vorderen Latzabschnitte und eine konvexe Krümmung der hinteren Latzabschnitte, erreicht werden, kann eine noch weiter verbesserte Anpassung an den Körper eines Benutzers insbesondere im Gesäßbereich erreicht werden.

Im Hinblick auf die Schaffung einer guten Passform und eines hohen Tragkomforts erweist es sich als vorteilhaft, wenn der Hüftgürtel mindestens einen elastischen Abschnitt, z.B. in jedem Seitenbereich einen elastischen Abschnitt umfasst, so dass der Hüftgürtel in Hüftumfangsrichtung elastisch dehnbar ist.

Weiterhin kann grundsätzlich vorgesehen sein, dass der Hüftgürtel im Bauchbereich und/oder im Rückenbereich im Wesentlichen undehnbar ausgebildet ist.

In einer alternativen Ausführungsform kann es sich jedoch auch als vorteilhaft erweisen, wenn sich ein oder mehrere elastische Abschnitte im Bauchbereich und/oder Rückenbereich des Hüftgürtels befinden, vorzugsweise in Form einer keilförmigen, trapezförmigen oder viereckigen Elastifizierung.

Eine besonders bevorzugte alternative Ausführungsform weist zwei trapezförmige Elastifizierungen im Rückenbereich des Hüftgürtels auf, die insbesondere als elastisch dehnbare Materialabschnitte ausgebildet sind, die jeweils in den Bereichen des Rückenbereichs des Hüftgürtels vorgesehen sind, die an die Seitenbereiche angrenzen und zwischen sich einen nicht elastischen Bereich einschließen. Dabei können die beiden parallelen Kanten der elastischen Materialabschnitte in Längsrichtung angeordnet sein und die beiden weiteren Kanten mit einer proximalen Querkante des Hüftgürtels und mit einer auf den Schrittbereich gerichteten Kante des Rückenbereichs zusammenfallen. Je nach Breite der elastischen Bereiche kann vorgesehen sein, dass diese sich in Längsrichtung bis in den Latzabschnitt, insbesondere den hinteren Latzabschnitt erstrecken.

Durch die Anordnung der elastischen Bereiches im Rückenbereich des Hüftgürtels wird der Sitz im Gesäßbereich insbesondere bei Bewegung des Benutzers verbessert und ein optimiertes Anliegen der Inkontinenzvorlage am Träger gewährleistet. Dies wird vorteilhafterweise durch die vorstehend beschriebene Ausgestaltung mit zwei trapezförmigen Elastifizierungen im Rückenbereich und ggf. im hinteren Latzabschnitt des Hüftgürtels noch verstärkt.

Besonders bevorzugt ist vorgesehen, dass die elastisch dehnbaren Materialabschnitte so ausgebildet sind, dass der absorbierende Kern zumindest hinsichtlich seiner Ränder der mit dem Hüftgürtel verbundenen Inkontinenzvorlage im ungedehnten und im gedehnten Zustand der elastischen Materialabschnitte im Bereich der Materialabschnitte liegt. Durch die Überlagerung des absorbierenden Kerns zumindest hinsichtlich seiner Ränder sowohl im gedehnten als auch im ungedehnten Zustand des Hüftgürtels und durch die im Rückenbereich des Hüftgürtels angeordneten elastischen Materialabschnitte, die zwischen sich einen nicht elastischen Bereich einschließen, wird eine ideale Paßform im Gesäßbereich gewährleistet und insbesondere eine Taschenbildung zwischen Gesäß und Inkontinenzvorlage sowie zwischen dem oberen, proximalen Querrand des Hüftgürtels und dem Verschlusselement am Latzabschnitt minimiert und somit ein gutes Anliegen des Produktes am Benutzer sichergestellt.

Dabei wird bei der bevorzugten Trapezform der elastischen Materialabschnitte die Zugspannung der elastischen Abschnitte in Anteile in Quer- und Anteil in Längsrichtung aufgeteilt, wodurch die Anpassung an die Gesäßform erreicht werden und ein seitliches Verrutschen der Inkontinenzvorlage und eine seitliche Taschenbildung minimiert werden können.

Weiter erweist es sich als vorteilhaft, wenn die Verschlusselemente des Latzabschnitts an einem schrittzugewandten Ende des Latzabschnitts vorgesehen sind. Darüber hinaus können am hinteren und/oder vorderen Latzabschnitt ein oder mehrere weitere Verschlusselemente vorgesehen sein, die zwischen dem schrittzugewandten Ende und dem Bauch- bzw. Rückenbereich positioniert sind und die der zusätzlichen Fixierung der Inkontinenzvorlage dienen.

Weiter erweist es sich als vorteilhaft, wenn die vorzugsweise mechanisch und/oder klebend wirkenden Verschlusselemente des Latzabschnitts, insbesondere am schrittzugewandten Ende des Latzabschnitts, auf einem streifenförmigen und quer zur Längsrichtung erstreckten Materialabschnitt vorgesehen sind, der an den Latzabschnitt angefügt ist. Auf diese Weise kann der Latzabschnitt über seine gesamte flächenhafte Erstreckung in Querrichtung in einer bestimmungsgemäßen zum Benutzer symmetrischen Weise an der Außenseite der Inkontinenzvorlage lösbar festgelegt werden. Der Latzabschnitt kann so weitgehend faltenfrei und wie erwähnt zu den Beinen und zum Schrittbereich bzw. zu einer auf den Benutzer abstellenden Längsmittelachse korrekt ausgebreitet und fixiert werden.

Vorzugsweise haben die mechanisch und/oder klebend wirkenden Verschlusselemente des Latzabschnitts eine Erstreckung in Querrichtung von 8 cm bis 14 cm, vorzugsweise 10 cm bis 12 cm, vorzugsweise 11 cm und vorzugsweise eine Erstreckung in Längsrichtung von 0,5 cm bis 5,0 cm, vorzugsweise 1,0 cm bis 4,0 cm, vorzugsweise von 1,0cm bis 3,0cm und vorzugsweise 1,5 cm. Damit ist eine ausreichend große Fläche für die Haftverbindung der mechanisch und/oder klebend wirkenden Verschlusselemente des Latzabschnitts mit der Außenseite der Inkontinenzvorlage für einen sicheren Produktsitz gewährleistet.

Die Außenseite der Inkontinenzvorlage ist dabei so auszubilden, dass sie mit den Verschlusselementen des Latzabschnittes korrespondiert, das heißt, bei einem mechanischen Verschlusselement in Form von Haken wird die Außenseite der Inkontinenzvorlage vorzugsweise durch eine schlaufenbildende Komponente, vorzugsweise durch ein Vlies, insbesondere die Vlieslage eines Vlies-Folien-Laminates, gebildet, während bei einem klebenden Verschlusselement die Außenseite der Inkontinenzvorlage vorzugsweise durch eine Folienlage gebildet wird, auf der die klebenden Verschlusselemente anhaften können.

Nach einem weiteren Erfindungsgedanken erweist es sich als vorteilhaft, wenn die Verschlusselemente des Latzabschnitts, insbesondere am schrittzugewandten Ende, vor der Ingebrauchnahme auf die körperzugewandte Seite des Latzabschnitts eingeschlagen und in dieser Konfiguration leicht lösbar an der körperzugewandten Seite des Latzabschnitts haftend sind. Auf diese Weise sind die an dem Latzabschnitt vorgesehenen, insbesondere mechanisch und/oder klebend wirkenden Verschlusselemente während des Anlegens des Hüftgürtels, insbesondere bei pflegebedürftigen Personen, so lange deaktiviert, wie sie nicht zum Festlegen der Inkontinenzvorlage benötigt werden. Sie behindern dabei nicht das Anlegen des Hüftgürtels, indem sie am Hüftgürtel oder an anderen Komponenten wie Bettlaken, Patientenunterlagen oder dergleichen, festhaken. In der Praxis kann es sich nämlich als vorteilhaft erweisen, wenn der vordere und/oder hintere Latzabschnitt auf sich selbst und/oder auf den Bauch- oder Rückenbereich gefaltet ist und die Faltung erst nach Anlegen des Hüftgürtels und der Inkontinenzvorlage geöffnet wird, um die Vorlage zu fixieren.

Zusätzlich kann diese Faltung der Latzabschnitte durch Fixierpunkte festgelegt werden. Dies kann zum Beispiel mittels einer Ultraschallverbindung, insbesondere kleiner separater Schweißpunkte erfolgen, die im Randbereich der überlappenden Lagen befestigt werden, aber auch mittels Klebeverbindungen, insbesondere Klebepunkten. Andere Arten der Anhaftung sind ebenfalls umfasst. Die Öffnungskraft dieser zusätzlichen Fixierung der Latzfaltung liegt signifikant unterhalb der Materialfestigkeit des Hüftgürtels und zerstört beim Auffalten der Latzabschnitte nicht das Material des Hüftgürtels.

Im Hinblick auf die praktische Handhabbarkeit erweist es sich auch als vorteilhaft, wenn der Hüftgürtel nur in einem Bereich öffenbar und schließbar und die Gürtelverschlusselemente in diesem Bereich an freien Endabschnitten des Hüftgürtels vorgesehen sind. Der Hüftgürtel ist also langgestreckt und hat zwei Endabschnitte, die mittels der Gürtelverschlusselemente aufeinander schließbar sind, um den Hüftgürtel um den Körperumfang des Benutzers herum anlegen zu können.

In Weiterbildung dieses Erfindungsgedankens erweist es sich als vorteilhaft, wenn im anderen Seitenbereich, in dem der Gürtel also nicht öffenbar und schließbar ist, Sekundärverschlusselemente vorgesehen sind, mittels derer eine Umfangslänge des Hüftgürtels einstellbar und so zumindest der vordere Latzabschnitt symmetrisch zum Schritt des Benutzers positionierbar ist. Vorzugsweise sind die Sekundärverschlusselemente so ausgebildet, dass sie im angelegten Zustand der Fixier- und Tragevorrichtung in der Ansicht optisch und/oder haptisch nicht von den Gürtelverschlusselementen unterscheidbar sind. Es kann sich jedoch auch als vorteilhaft erweisen, wenn die Sekundärverschlusselemente von den Gürtelverschlusselementen optisch und/oder haptisch unterscheidbar sind, um auf die unterschiedliche Funktion der Verschlusselemente hinzuweisen. Dies kann beispielsweise durch unterschiedliche Farben, Formen, Textur oder auf andere Weise erfolgen. Die Gürtelverschlusselemente und die Sekundärverschlusselemente sind dabei vorteilhafter Weise beidseits des Bauchbereichs und/oder in Seitenbereichen des Hüftgürtels vorgesehen.

Beim Anlegen des Hüftgürtels führt die Pflegeperson den geöffneten langgestreckten Hüftgürtel bei einem liegenden Patienten unter dessen Körper auf Hüft- oder Rückenhöhe hindurch. Sodann wird der Gürtel zur Bildung der geschlossenen Hüftöffnung mittels der Gürtelverschlusselemente auf sich selbst geschlossen. In diesem Zustand ist der Gürtel zwar angelegt, jedoch noch nicht durch Aktivierung der Sekundärverschlusselemente in seine optimale Passform gebracht. Beispielsweise wird in diesem Zustand der bauchseitige Latzabschnitt durch den Zug in Hüftumfangsrichtung, der durch das Schließen der Gürtelverschlusselemente ausgeübt wird, zu der betreffenden Seite hin gezogen. Dem kann nun durch Einstellen der optimalen Hüftumfangslänge und einer geeigneten Spannung in Hüftumfangsrichtung begegnet werden, indem die Sekundärverschlusselemente entsprechend optimal eingestellt werden. Mittels dieser Sekundärverschlusselemente werden also - wie bereits ausgeführt - nicht zwei offene freie Enden aufeinander geschlossen, sondern es wird die Umfangslänge des Hüftgürtels gegebenenfalls verstellt.

Die Sekundärverschlusselemente können in verschiedener Weise verwirklicht sein. Nach einer bevorzugten Ausführungsform der Erfindung wird vorgeschlagen, dass eine Komponente der Sekundärverschlusselemente auf einem an den Hüftgürtel angefügten Materialabschnitt vorgesehen ist und die andere Komponente an einer Außenseite des Hüftgürtels selbst vorgesehen ist. Solchenfalls wird also der Hüftgürtel als solcher nicht verändert, sondern er erhält ein zusätzliches Element in Form des die Sekundärverschlusselemente tragenden Materialabschnitts, der in an sich beliebiger Weise auf das Material des Hüftgürtels aufgebracht werden kann, beispielsweise durch Aufnähen, Aufkleben, Aufsiegeln, Ultraschallschweißen oder dergleichen übliche Fügeverfahren.

Es erweist sich auch als vorteilhaft, wenn sich die Gürtelverschlusselemente und/oder die Sekundärverschlusselemente über im Wesentlichen die gesamte Erstreckung des Hüftgürtels in Längsrichtung, also über die gesamte Breite des Hüftgürtels, erstrecken. Auf diese Weise kann beim Einstellen der Sekundärverschlusselemente ein gleichmäßiger Zug über die gesamte Breite des Gürtels auf den Gürtel ausgeübt werden.

Es ist auch denkbar, dass sich die Gürtelverschlusselemente und/oder die Sekundärverschlusselemente zu ihrem freien Ende hin verjüngen oder verschmälern, um ein Reiben der Ecken dieser Verschlusselemente auf der Haut des Benutzers zu minimieren.

In einer alternativen Ausführungsform ist es darüber hinaus denkbar, dass die Gürtelverschlusselemente und/oder die Sekundärverschlusselemente sich nicht über im Wesentlichen die gesamte Breite des Hüftgürtels erstrecken. In einem solchen Fall ist es besonders vorteilhaft, wenn die Quererstreckung der Gürtelverschlusselemente und/oder der Sekundärverschlusselemente größer ist als ihre Längserstreckung.

Vorzugsweise stehen die Gürtelverschlusselemente und/oder die Sekundärverschlusselemente über den Seitenrand des Hüftgürtels um mindestens 30 mm, insbesondere um mindestens 40 mm und weiter insbesondere um mindestens 50 mm in Querrichtung über. Die Erstreckung der Gürtelverschlusselemente und/oder die Sekundärverschlusselemente in Längsrichtung der Fixier- und Tragevorrichtung beträgt insbesondere mindestens 25 mm, weiter insbesondere mindestens 35 mm und weiter insbesondere mindestens 45 mm. Damit ergibt sich ein bevorzugtes Verhältnis von Quererstreckung zu Längserstreckung von 30 zu 25, vorzugsweise von 40 zu 35 und insbesondere von 50 zu 45. Insbesondere kann vorgesehen sein, dass die Gürtelverschlusselemente und/oder die Sekundärverschlusselemente trapezförmig ausgebildet sind, insbesondere mit einem Seitenverhältnis von 50mm in Querrichtung zu 35/30mm in Längsrichtung, wobei die kürzere Seite des Trapezes die freie Seite der Verschlusselemente bildet. Damit ist eine ausreichend große Fläche für die Haftverbindung der Gürtelverschlusselemente und/oder der Sekundärverschlusselemente mit der Außenseite des Hüftgürtels für einen sicheren Produktsitz gewährleistet.

Dabei können die Gürtelverschlusselemente und/oder die Sekundärverschlusselemente sogenannte Anfass- oder Griffabschnitte umfassen oder mit diesen verbunden sein. Die Anfass- oder Griffabschnitte sind vorzugsweise am freien Ende der Gürtel- oder Sekundärverschlusselemente vorgesehen und weisen keine Verschlussmittel z.B. in Form von mechanischen und/oder klebenden Elementen auf. Hierdurch wird die Anfassbarkeit der Verschlussmittel verbessert.

In Weiterbildung der Erfindung wird vorgeschlagen, die Gürtelverschlusselemente und/oder die Sekundärverschlusselemente zumindest abschnittsweise elastisch auszubilden.

Zum Verkürzen der Umfangslänge des Gürtels unter Verwendung der Sekundärverschlusselemente wird dabei vorzugsweise eine Z-förmige Faltung des Gürtels durch Schließen der Sekundärverschlusselemente bewirkt, die durch den dabei entstehenden Druck des Hüftgürtels gegen die Körperoberfläche des Benutzers und/oder durch Reibung der aneinander anliegenden Abschnitte der Z-förmigen Faltung gehalten wird.

Im Hinblick auf die Bemessung des Hüftgürtels erweist es sich als vorteilhaft, wenn der Hüftgürtel an vorzugsweise jeder Stelle eine Erstreckung in Längsrichtung von wenigstens 5 cm, insbesondere von wenigstens 6 cm, insbesondere von wenigstens 7 cm, insbesondere von wenigstens 8 cm aufweist. Hierbei ist die Abmessung des Hüftgürtels in Längsrichtung, also die Gürtelbreite quer zu seiner Umfangserstreckung, und zwar außerhalb des vorderen oder hinteren Latzabschnitts gemeint. Die Erstreckung in Längsrichtung ist vorzugsweise höchstens 25 cm. Besonders bevorzugt ist die Erstreckung im Bauchbereich geringer als im Rückenbereich, und der obere Rand des Hüftgürtels ist vorzugsweise leicht konturiert, um einen Bauchausschnitt zu bilden, der die Passform des Produkts verbessert sowie ein Rollen oder Umschlagen des Gürtels im Bauchbereich verhindert.

Hinsichtlich der Bemessung des Latzabschnitts erweist es sich als vorteilhaft, wenn der Latzabschnitt eine Erstreckung in Längsrichtung über eine schrittzugewandten distale Querkante des Bauch- oder Rückenbereichs in distaler Richtung auf den Schrittbereich von wenigstens 5 cm, insbesondere von wenigstens 6 cm, insbesondere von wenigstens 7 cm, insbesondere von wenigstens 8 cm, insbesondere von wenigstens 9 cm, insbesondere von wenigstens 10 cm, insbesondere von höchstens 20 cm, insbesondere von 10 - 18 cm, insbesondere von 10-16 cm aufweist, wobei der vordere und hintere Latzabschnitt die gleiche oder eine unterschiedliche Erstreckung haben können. Die Erstreckung des Hüftgürtels in Längsrichtung beträgt im Bereich des Latzabschnitts vorne bei konturiertem Bauchausschnitt vorzugsweise 13 cm bis 19 cm, vorzugsweise 15 cm bis 17 cm, insbesondere 16 cm, im Bereich des Latzabschnitts hinten vorzugsweise 20 cm bis 27 cm, vorzugsweise 22 cm bis 25 cm, insbesondere 23,5 cm. Bei konturiertem Bauchausschnitt wurde dieser Wert ausgehend vom distalsten (tiefsten) Punkt der proximalen Querkante des Bauchbereichs bestimmt.

Die Erstreckung des Hüftgürtels in Querrichtung, das heisst, in Hüftumfangsrichtung beträgt zwischen 75 und 175 cm , vorzugsweise für die Größe S zwischen 50 und 90 cm, für die Größe M zwischen 80 und 120 cm, für die Größe L zwischen 100 und 140 cm, für die Größe XL zwischen 120 und 160 cm sowie für die Größe XXL zwischen 140 und 190 cm und orientiert sich an den Kleiderkonfektionsgrößen.

Nach einer bevorzugten Ausführungsform der Erfindung ist der betreffende Latzabschnitt einstückig mit dem Bauchbereich und/oder mit dem Rückenbereich des Hüftgürtels ausgebildet. Separat angestückte Latzabschnitte aus dem gleichen Material wie der übrige Hüftgürtel oder aus einem anderen Material als der übrige Hüftgürtel sind aber ebenfalls denkbar.

Der Bauchbereich und/oder Rückenbereich des Hüftgürtels ist vorzugsweise von einem Vliesmaterial oder Vliesverbundmaterial, insbesondere von einem im Wesentlichen undehnbaren Vliesmaterial oder Vliesverbundmaterial, gebildet.

Weiter ist es möglich, wenn ein elastisch dehnbarer Seitenbereich des Hüftgürtels mit einem im wesentlichen undehnbaren Bauchbereich und einem im Wesentlichen undehnbaren Rückenbereich unlösbar verbunden ist. Beispielsweise umfasst jeder Seitenbereich einen elastisch dehnbaren Abschnitt. In einer alternativen besonders bevorzugten Ausführungsform kann es sich als vorteilhaft erweisen, wenn Teile des Rückenbereichs, insbesondere die seitlichen Teile des Rückenbereichs, die an die Seitenbereiche angefügt sind, elastische ausgebildet sind. Die elastischen und unelastischen Teile können vorzugsweise unlösbar miteinander verbunden sein.

Darüber hinaus erweist es sich als vorteilhaft, wenn der vordere und/oder hintere Latzabschnitt farbig ausgebildet ist, wobei die Abschnitte vorzugsweise unterschiedlich farbig sind, um als visuelle Anlegehilfe zu dienen.

Weiterhin ist es vorteilhaft, wenn auf dem vorderen und/oder hinteren Latzabschnitt eine Markierung angebracht ist, die als Positionierhilfe für die Inkontinenzvorlage dient, derart, dass bei korrekt angelegter Inkontinenzvorlage und Fixier- und Tragevorrichtung die Markierung auf dem vorderen und/oder hinteren Latzabschnitt mit einer auf der äußeren Lage der Inkontinenzvorlage sichtbaren Markierung in Einklang gebracht ist. Beispielsweise kann eine Ausnehmung mittig am schrittzugewandten Rand des vorderen und/oder hinteren Latzabschnitts in Einklang gebracht werden mit einer Markierung z.B. in Form eines in Längsrichtung mittig aufgebrachten Nässeindikators auf der äußeren Lage der Inkontinenzvorlage. Alternativ kann die Breite des unteren Rand des vorderen und/oder hinteren Latzabschnitts herstellerseitig so gewählt werden, dass diese mit dem Abstand von Markierungen auf der äußeren Lage der Inkontinenzvorlage, wie beispielsweise einer Größen- oder Saugstärken-Markierung, in Einklang gebracht werden kann.

Darüber hinaus betrifft die Erfindung ein System aus einer Fixier- und Tragevorrichtung der zuvor beschriebenen Art mit einer daran lösbar festlegbaren Inkontinenzvorlage mit einer Längsrichtung und einer Querrichtung sowie zwei Längskanten und zwei Querkanten sowie einem Rücken- und einem Bauchbereich, die durch die Breite der Inkontinenzvorlage in Querrichtung sowie die Erstreckung von einer Querkante der Inkontinenzvorlage bis zu einer distalen Kante eines Latzabschnitts des Hüftgürtels in Längsrichtung definiert ist, wobei der Rückenbereich der angelegten Inkontinenzvorlage durch den hintere Rückenbereich des Hüftgürtels und/oder den hinteren Latzabschnitt zu mindestens 50% überdeckt ist, insbesondere zu mindestens 60% und insbesondere zu mindestens 70%.

Weiterhin bevorzugt ist eine entsprechende Überdeckung im Bauchbereich der Inkontinenzvorlage von mindestens 50%, insbesondere mindestens 60% und bevorzugt mindestens 70% durch den Bauchbereich des Hüftgürtels und/oder den hiermit verbundenen Latzabschnitt.

Dabei ist bei dem System aus Fixier- und Tragevorrichtung und Inkontinenzvorlage vorgesehen, dass die Inkontinenzvorlage zwei Längs- und zwei Querkanten umfasst und im an der Fixier- und Tragevorrichtung festgelegten Zustand, die Querkanten im wesentlichen mit einer Querkante, insbesondere der dem Schrittbereich abgewandten, also proximalen Querkante des Hüftgürtels zusammenfallen.

Besonders bevorzugt ist vorgesehen bei der Ausgestaltung des erfindungsgemäßen Systems, dass der Hüftgürtel im hinteren Rückenbereich und/oder dem hiermit verbundenen Latzabschnitt elastisch dehnbare Materialabschnitte aufweist, wobei die elastisch dehnbaren Materialabschnitte insbesondere an die Seitenbereiche anschließen und zwischen sich einen nicht elastisch dehnbaren Abschnitt des Rückenbereichs und/oder des hiermit verbundenen Latzabschnitts einschließen, wobei die elastisch dehnbaren Materialabschnitte insbesondere trapezförmig ausgebildet sind und wobei die elastisch dehnbaren Materialabschnitte so ausgebildet sind, dass der absorbierende Kern zumindest der mit dem Hüftgürtel verbundenen Inkontinenzvorlage zumindest hinsichtlich seiner Ränder im ungedehnten und im gedehnten Zustand der Materialabschnitte im Bereich der Materialabschnitte liegt.

Schließlich betrifft die Erfindung nach einem selbständigen Erfindungsgedanken Fixer- und Tragevorrichtung für eine wegwerfbare absorbierende Inkontinenzvorlage mit einem mittels Gürtelverschlusselementen auf sich selbst lösbar schließbaren und so eine in Hüftumfangsrichtung durchgehende Hüftöffnung bildenden Hüftgürtel, an den die Inkontinenzvorlage lösbar anbringbar ist, so dass sie im Schrittbereich des Benutzers getragen und nach Gebrauch wieder vom Hüftgürtel entfernt und verworfen werden kann, wobei der Hüftgürtel einen vorderen Bauchbereich, einen hinteren Rückenbereich und einen linken und rechten Seitenbereich umfasst, der Hüftgürtel ausgehend von dem Rückenbereich und dem Bauchbereich je einen sich in einer Längsrichtung in Richtung auf den Schrittbereich des Benutzers erstreckenden Latzabschnitt aufweist, der an seiner körperzugewandten Seite Verschlusselemente aufweist, die mit komplementären Verschlusselementen auf der körperabgewandten Seite der Inkontinenzvorlage lösbar haftend zusammenwirken, wobei die Inkontinenzvorlage einen Rückenbereich und einen Bauchbereich aufweist deren Abmessungen durch die Breite der Inkontinenzvorlage quer zur Längsrichtung sowie die Erstreckung in Längsrichtung von einer Querkante der Inkontinenzvorlage bis zu einer distalen Kante des die Inkontinenzvorlage überdeckenden vorderen oder hinteren Latzabschnitts bestimmt ist, wobei der Hüftgürtel im hinteren Rückenbereich und/oder dem hiermit verbundenen Latzabschnitt elastisch dehnbare Materialabschnitte aufweist und die elastisch dehnbaren Materialabschnitte so ausgebildet sind, dass ein absorbierender Kern, wenigstens hinsichtlich seiner Ränder, der mit dem Hüftgürtel verbundenen Inkontinenzvorlage im ungedehnten und im gedehnten Zustand der Materialabschnitte im Bereich der Materialabschnitte liegt.

Eine solche selbständige Erfindung kann darüber hinaus die weiteren vorstehenden Merkmale zur zuerst genannten Erfindung umfassen.

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform der erfindungsgemäßen Fixier- und Tragevorrichtung für eine wegwerfbare absorbierende Inkontinenzvorlage.

In der Zeichnung zeigt:
Figuren 1 und 2 eine Ausführungsform der erfindungsgemäßen Fixier- und Tragevorrichtung im angelegten Zustand zusammen mit einer wegwerfbaren absorbierenden Inkontinenzvorlage;
Figur 3 eine schematische Draufsicht auf die Fixier- und Tragevorrichtung im geöffneten und auf eine ebene Unterlage ausgebreiteten Zustand und
Figur 4 eine schematische Darstellung einer Fixier- und Tragevorrichtung gemäß der Erfindung mit daran festgelegter Inkontinenzvorlage im Rückenbereich;
Figur 5 eine schematische Darstellung einer Fixier- und Tragevorrichtung gemäß der Erfindung mit daran festgelegter Inkontinenzvorlage im Bauchbereich;
Figur 6 eine Darstellung gemäß Figur 4 elastischen Materialabschnitten im Rückenbereich und im hinteren Latzabschnitt der Fixier- und Tragevorrichtung;
Figur 7 eine schematische Darstellung des Rückenbereichs und des hinteren Latzabschnitts der Fixier- und Tragevorrichtung.

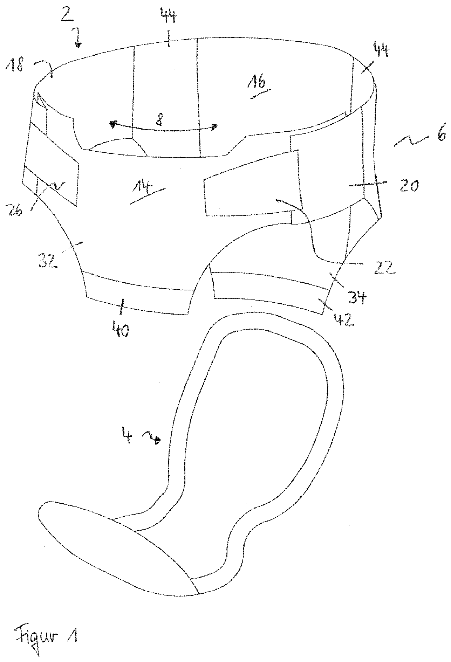

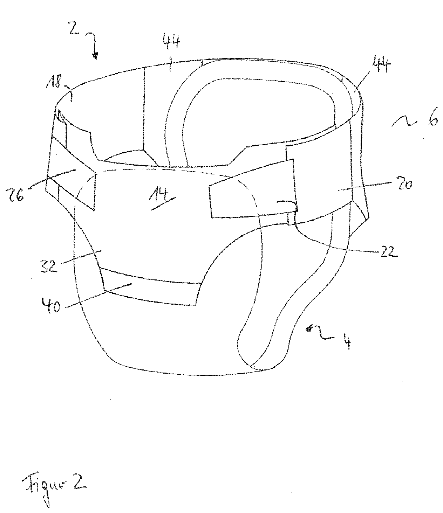

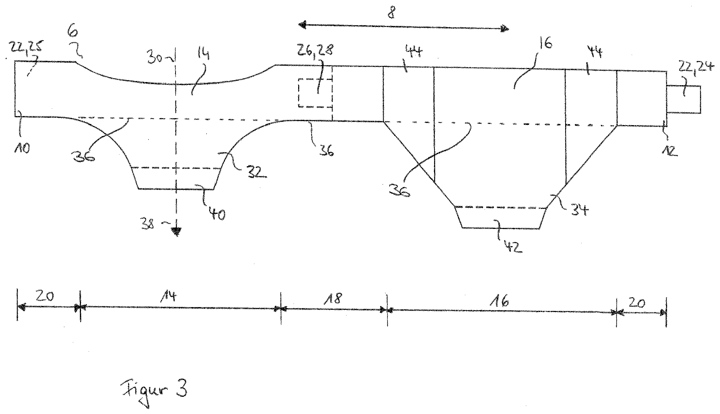

Die Figuren 1 und 2 zeigen perspektivische Ansichten einer insgesamt mit dem Bezugszeichen 2 bezeichneten Fixier- und Tragevorrichtung für eine mit Bezugszeichen 4 angedeutete absorbierende Inkontinenzvorlage, und zwar im an einen Benutzer angelegten Zustand. Die Fixier- und Tragevorrichtung 2 umfasst einen auf sich selbst lösbar schließbaren Hüftgürtel 6, der sich entlang einer Hüftumfangsrichtung 8 erstreckt und zwei freie Endabschnitte 10, 12 (s. Figur 3) aufweist, die zur Bildung der geschlossenen Hüftumfangsform auf sich selbst schließbar sind.

Die Fixier- und Tragevorrichtung 2 bzw. ihr Hüftgürtel 6 umfassen einen vorderen Bauchbereich 14, einen hinteren Rückenbereich 16 sowie einen linken Seitenbereich 18 und einen rechten Seitenbereich 20.

Zum Schließen des Hüftgürtels 6 sind an dessen freien Endabschnitten 10, 12 Gürtelverschlusselemente 22 vorgesehen, die im bevorzugt und beispielhaft dargestellten Fall von mechanisch wirkenden Verschlusselementen in Form eines Haken/Schlaufenverschlusssystems gebildet sind. Dabei ist in den Figuren mit Bezugszeichen 24 die hakenförmige Verschlusskomponente dargestellt, die über den Seitenrand 12 des Hüftgürtels hinausragt und mit einer schlaufenbildenden Komponente 25 in Form der äußeren Oberfläche des Bauchbereichs 14 lösbar haftend zusammenwirkt. Beispielsweise weist die Außenseite des Bauchbereichs 14 ein Vliesmaterial auf, welches die schlaufenförmige Komponente 25 des Verschlusssystems bildet.

Des Weiteren weist der nur an seinen Endabschnitten 10, 12, also nur an einer Stelle öffenbare Hüftgürtel 6 Sekundärverschlusselemente 26 auf, mittels derer die Umfangslänge des Hüftgürtels 6 einstellbar ist. Die Sekundärverschlusselemente 26 sind seitlich am Bauchbereich 14 bzw. am linken Seitenbereich 18 so vorgesehen, dass sie im angelegten Zustand der Fixier- und Tragevorrichtung (s. Figur 1) denselben optischen Eindruck wie die Gürtelverschlusselemente 22 hinsichtlich Ausbildung und Anordnung vermitteln, obschon ihre Funktion verschieden ist. Mittels der Sekundärverschlusselemente 26 werden nämlich keine öffenbaren und voneinander trennbaren Endabschnitte des Hüftgürtels 6 miteinander verbunden, sondern die Sekundärverschlusselemente 26 dienen lediglich dazu, den bestimmungsgemäßen Sitz des Hüftgürtels 6 an die Tragesituation anzupassen. Hierfür umfassen die Sekundärverschlusselemente 26 einen auf die Außenseite des Hüftgürtels 6 aufgebrachten Materialabschnitt 28, auf dem oder an dem sie selbst vorgesehen sind. Bei den Sekundärverschlusselementen 26 handelt es sich wiederum in bevorzugter Weise um mechanisch wirkende Verschlusselemente, insbesondere um Haken/Schlaufenmaterialien. Im beispielhaft dargestellten und bevorzugten Fall sind die Sekundärverschlusselemente 26 derart ausgebildet, dass an dem Materialabschnitt 28 eine hakenbildende Komponente des Verschlusssystems vorgesehen ist, die mit einer schlaufenbildenden Außenseite des Bauchbereichs 14 zusammenwirkt, so wie dies vorausgehend bei den Gürtelverschlusselementen 22 beschrieben wurde.

Als weitere wesentliche Komponente umfasst der Hüftgürtel 6 ausgehend vom Bauchbereich 14 und vom Rückenbereich 16 je einen symmetrisch zu einer Längsmittelachse 30 ausgebildeten vorderen und hinteren Latzabschnitt 32, 34. Die Latzabschnitte 32, 34 sind im beispielhaft bevorzugt dargestellten Fall einstückig mit dem Hüftgürtel 6 ausgebildet; sie erstrecken sich von einer schrittzugewandten oder unteren, distalen Querkante 36 des Hüftgürtels entlang der Längsmittelachse 30 in Richtung 38 auf den Schrittbereich. Sie können sich in Richtung auf den Schrittbereich verjüngen und seitlich bogenförmig konturiert ausgebildet sein. An ihrem unteren, schrittzugewandten Endbereich umfassen die Latzabschnitte 32, 34 mechanisch wirkende Verschlusselemente 40, 42, und zwar auf der körperzugewandten Seite. Diese mechanisch wirkenden Verschlusselemente 40, 42 wirken lösbar haftend mit komplementären mechanisch wirkenden Verschlusselementen auf der körperabgewandten Seite der Inkontinenzvorlage 4 zusammen, um die Inkontinenzvorlage 4 lösbar, jedoch verliersicher an der Fixier- und Tragevorrichtung 2 festzulegen. Bevorzugtermaßen handelt es sich bei den Verschlusselementen 40, 42 um die hakenbildende Komponente eines mechanisch wirkenden Verschlusssystems, die mit der schlaufenbildenden Komponente auf der körperabgewandten Seite der Inkontinenzvorlage 4 zusammenwirkt. Die schlaufenbildende Komponente kann dabei in vorteilhafter Weise von einer Vliesbeschichtung, vorzugsweise der Vlieslage eines Vlies-Folien-Laminates, der Inkontinenzvorlage 4 gebildet sein.

Der Hüftgürtel 6 ist vorzugsweise aus Vlies- oder Vliesverbundmaterialien hergestellt. Im beispielhaft dargestellten jedoch bevorzugten Fall sind der Bauchbereich 14 und die beiden Seitenbereiche 18, 20 aus einem im Wesentlichen undehnbaren Vliesmaterial oder Vliesverbundmaterial gebildet, dessen Außenseite als schlaufenbildende Komponente für die Gürtelverschlusselemente 22 bzw. die Sekundärverschlusselemente 26 dient. Der Rückenbereich 16 ist zumindest teilweise elastisch dehnbar ausgebildet, indem er ein Paar elastisch dehnbare Materialabschnitte 44 aufweist, die bestimmungsgemäß unlösbar mit dem undehnbaren Material der Seitenbereiche 18, 20 verbunden sind und zwischen sich einen elastisch undehnbaren Bereich des Rückenbereichs einschließen.

Das Anlegen der Fixier- und Tragevorrichtung 2 zusammen mit der absorbierenden Inkontinenzvorlage 4 geschieht folgendermaßen: Zunächst wird der Hüftgürtel 6 bei bettlägerigen pflegebedürftigen Patienten durch eine Pflegeperson unter dem Körper des Patienten auf Hüfthöhe hindurchgeführt. Der Hüftgürtel 6 wird dabei so positioniert, dass der Rückenbereich 16 unterhalb des Gesäßes und der Bauchbereich 14 ungefähr vorne mittig zu liegen kommen. Sodann werden die freien Endabschnitte 10, 12 des Hüftgürtels 6 übereinander positioniert und mittels der Gürtelverschlusselemente 22 aufeinander geschlossen. Durch anschließendes Positionieren und Schließen des Materialabschnitts 28 und der Sekundärverschlusselemente 26 wird die Länge und Spannung des Hüftgürtels 6 in Umfangsrichtung 8 optimiert, so dass ein gleichmäßiger Zug auf den Bauchbereich 14 links und rechts ausgeübt wird und der Bauchbereich 14 wie auch der Rückenbereich 16 vorzugsweise symmetrisch in Bezug auf den Schrittbereich des Patienten und möglichst faltenfrei zu liegen kommen. Hierbei ist auch auf ein angenehmes Traggefühl abzustellen. Sodann wird der vordere und hintere Latzabschnitt 32, 34 mit der zuvor oder erst jetzt im Schrittbereich des Patienten positionierten Inkontinenzvorlage 4 unter Verwendung der mechanisch wirkenden Verschlusselemente 40, 42 lösbar haftend verbunden. Auch hierbei ist darauf zu achten, dass die vorderen und hinteren Latzabschnitte 32, 34 unter Ausübung einer gleichmäßigen und moderaten Zug- bzw. Haltekraft auf die Inkontinenzvorlage 4 fixiert werden, damit die Inkontinenzvorlage 4 in ihrer bestimmungsgemäßen symmetrischen Anordnung im Schrittbereich des Patienten verbleibt.

Hinsichtlich der Reihenfolge der vorausgehend geschilderten Schritte zum Anlegen des Hygieneartikels bzw. Inkontinenzsystems ist die Pflegeperson verhältnismäßig frei. Beispielsweise kann es sich in bestimmten Pflegesituationen als vorteilhaft erweisen, dass zunächst die Inkontinenzvorlage im Schrittbereich des Patienten vorpositioniert wird und erst dann der Hüftgürtel 6 auf sich selbst geschlossen wird. Es kann sich auch als zweckmäßig erweisen, dass die Sekundärverschlusselemente 26 erst nach dem Verbinden der Latzabschnitte 32, 34 mit der Inkontinenzvorlage 4 genutzt werden, um die optimale Spannung in Hüftumfangsrichtung 8 vorzugeben.

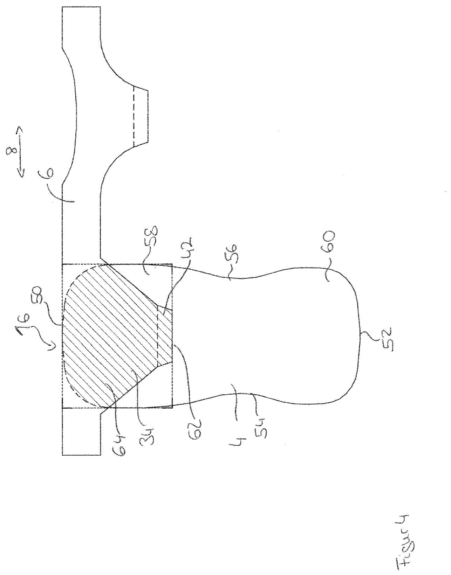

Figur 4 zeigt einen erfindungsgemäßen Hüftgürtel 6 in schematischer Darstellung, wobei hier eine Inkontinenzvorlage 4 im Bereich des hinteren Rückenbereichs 16 sowie des hinteren Latzabschnitts 34 mit dem Hüftgürtel 6 verbunden gezeigt ist. Die Inkontinenzvorlage 4 umfasst dabei zwei Querkanten 50 und 52 sowie zwei Längskanten 54 und 56, wobei die Längskanten 54 und 56 konturiert ausgebildet sein können, um sich einem Benutzer der Inkontinenzvorlage 4 anzupassen und so im Schrittbereich einen geringeren Abstand voneinander aufweisen.

Darüber hinaus umfasst die Inkontinenzvorlage 4 einen Rückenbereich 58 sowie einen Bauchbereich 60. Der Rückenbereich 58 wird dabei durch die Breite der Inkontinenzvorlage in Querrichtung, wobei die Querrichtung der Hüftumfangsrichtung 8 entspricht, sowie in Längsrichtung durch die Querkante 50 der Inkontinenzvorlage 4 und das distale Ende des Latzabschnitts 62 definiert.

Dieser Rückenbereich 58 der Inkontinenzvorlage wird erfindungsgemäß zu 50% durch den Hüftgürtel 6 in diesem Bereich gebildet durch den hinteren Rückenbereich 16 sowie den hiermit verbundenen Latzabschnitt 34 einschließlich des hiermit verbundenen Verschlusselements 42 überdeckt. Die überdeckte Fläche ist hier mit dem Bezugszeichen 64 versehen und schraffiert dargestellt. Die Überdeckung der schraffierten Fläche beträgt mindestens 50%, vorzugsweise mindestens 60% und besonders vorzugsweise mindestens 70%.

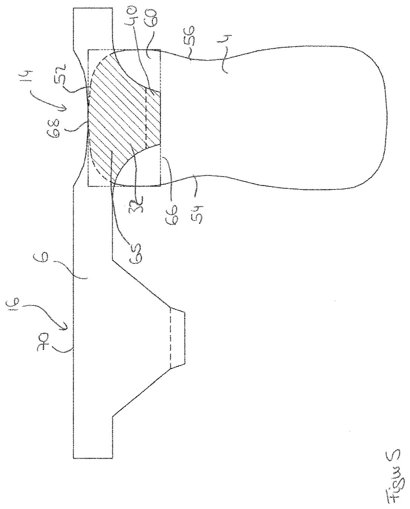

Figur 5 zeigt nun eine analoge Gestaltung für den vorderen Bauchbereich 60 der Inkontinenzvorlage 4, wobei im übrigen gleiche Bauteile mit gleichen Bezugszeichen versehen sind. Der vordere Bauchbereich 60 der Inkontinenzvorlage 4 wird durch die Querkante 52 der Inkontinenzvorlage sowie hinsichtlich der Breite durch die beiden Längskanten 54 und 56 der Inkontinenzvorlage 4 in diesem Bereich definiert. Die Begrenzung in distaler Richtung wird durch die schrittzugewandte Kante 66 des Latzabschnitts 32 gebildet. Auch hier soll ebenfalls eine Überdeckung des Bauchbereichs 60 der Inkontinenzvorlage 4 durch den vorderen Bauchbereich 14 des Hüftgürtels 6 sowie den Latzabschnitt 32 einschließlich des Verschlusselementes 40 von mindestens 50% gegeben sein. Die überdeckte Fläche ist mit 65 gekennzeichnet und schraffiert dargestellt.

Dabei wird die Inkontinenzvorlage 4 bevorzugt so angelegt, dass ihre oberen Querkanten 50 und 52 im mit dem Hüftgürtel 6 verbundenen Zustand jeweils mit der proximalen Querkante 68 des Bauchbereichs 14 des Hüftgürtels 6 bzw. der proximalen Querkante 70 des Rückenbereichs 16 des Hüftgürtels 6 zusammenfallen.

Die vorgenannte Überdeckung wird jedoch auch erzielt, wenn die Inkontinenzvorlage 4 nicht bündig mit dem Hüftgürtel 6 abschließt, sondern in proximaler Richtung ein Stück darüber hinausragt oder die Querkante des Hüftgürtels 6 in proximaler Richtung über die Querkante der Inkontinenzvorlage 4, insbesondere im Rückenbereich hinausragt.

Im Falle einer konturierten proximalen Querkante 68, 70 des Hüftgürtels, beispielsweise durch eine Konturierung im Bauchbereich 14 im Sinne eines Bauchausschnitts, läuft die proximale Kante 68 des Bauchbereichs 14 parallel zur Hüftumfangsrichtung durch den distalsten Punkt dieser Konturierung.

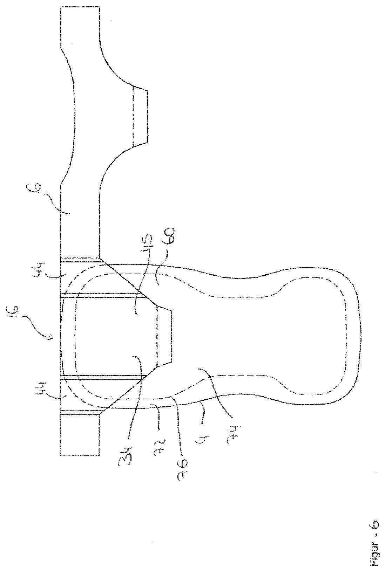

Figur 6 zeigt nun eine analoge Gestaltung zu Figur 4, wobei hier hinsichtlich der Inkontinenzvorlage 4 neben der äußeren Schicht der Rückenschicht (Backsheet) 72 auch der absorbierende Kern 74 gestrichelt dargestellt gezeigt ist ebenso wie die elastischen Abschnitte 44 die trapezförmig ausgestaltet im Rückenbereich 16 des Hüftgürtels 6 angeordnet sind und zwischen sich einen nicht elastischen Abschnitt 45 einschließen. Die elastischen Abschnitte 44 erstrecken sich dabei bis in den Latzabschnitt 34 hinein.

Es ist dabei erfindungsgemäß vorgesehen, dass die äußere Kante 76 des absorbierenden Kerns 74 sowohl im gedehnten als auch im ungedehnten Zustand der elastischen Materialabschnitte 44 durch diese überfangen wird. Das heißt, die äußere Kante 76 fällt stets in den Bereich der elastischen Materialabschnitte 44. Auf diese Weise wird erreicht, dass eine besonders gute Passform der angelegten Inkontinenzvorlage am Hüftgürtel 6 im Gesäßbereich erzielt werden kann, insbesondere zusammen mit der Abdeckung des Rückenbereichs 58 der Inkontinenzvorlage 4 durch die entsprechenden Bereiche des Hüftgürtels 6 von mindestens 50%. Dadurch kann eine Taschenbildung im Gesäßbereich verhindert werden, die Passform verbessert und somit die Gefahr eines Auslaufen sicherer reduziert werden.

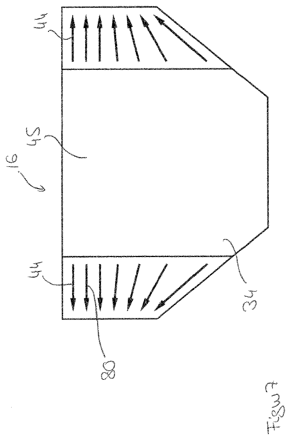

Figur 7 zeigt nun den hinteren Rückenbereich 16 eines Hüftgürtels 6 mit den elastischen Bereichen 44, die zwischen sich den unelastischen Bereich 45 einschließen. Darüber hinaus ist auch der Latzabschnitt 34 dargestellt. Mit dem Bezugszeichen 80 sind die Richtungen der Zugspannung gezeigt, wobei gut erkannt werden kann, dass durch die trapezförmige Form der Abschnitte 44 die Kraft in quer und längs verlaufende Anteile, insbesondere im distalen Bereich aufgeteilt wird, wodurch eine optimale Anpassung an die Gesäßform erreicht wird.

## Patentansprüche

1. Fixer- und Tragevorrichtung (2) für eine wegwerfbare absorbierende Inkontinenzvorlage (4) mit einem mittels Gürtelverschlusselementen (22) auf sich selbst lösbar schließbaren und so eine in Hüftumfangsrichtung (8) durchgehende Hüftöffnung bildenden Hüftgürtel (6), an den die Inkontinenzvorlage (4) lösbar anbringbar ist, so dass sie im Schrittbereich des Benutzers getragen und nach Gebrauch wieder vom Hüftgürtel (6) entfernt und verworfen werden kann, wobei der Hüftgürtel (6) einen vorderen Bauchbereich (14), einen hinteren Rückenbereich (16) und einen linken und rechten Seitenbereich (18, 20) umfasst, der Hüftgürtel (6) ausgehend von dem Rückenbereich (16) und dem Bauchbereich (14) je einen sich in einer Längsrichtung in Richtung (38) auf den Schrittbereich des Benutzers erstreckenden Latzabschnitt (32, 34) aufweist, der an seiner körperzugewandten Seite Verschlusselemente (40, 42) aufweist, die mit komplementären Verschlusselementen auf der körperabgewandten Seite der Inkontinenzvorlage (4) lösbar haftend zusammenwirken, wobei die Inkontinenzvorlage (4) einen Rückenbereich (58) und einen Bauchbereich (60) aufweist deren Abmessungen durch die Breite der Inkontinenzvorlage (4) quer zur Längsrichtung sowie die Erstreckung in Längsrichtung von einer Querkante (50,52) der Inkontinenzvorlage (4) bis zu einer distalen Kante (62,66) des die Inkontinenzvorlage (4) überdeckenden vorderen oder hinteren Latzabschnitts (32,34) bestimmt ist, **dadurch gekennzeichnet, dass** der Rückenbereich (58) der angelegten Inkontinenzvorlage (4) durch den hintere Rückenbereich (16) des Hüftgürtels und den hinteren Latzabschnitt (34) zu mindestens 50% überdeckt ist und der Latzabschnitt eine Erstreckung in Längsrichtung über eine schrittzugewandten distale Querkante des Bauch- oder Rückenbereichs in distaler Richtung auf den Schrittbereich von wenigstens 5 cm, insbesondere von wenigstens 6 cm, insbesondere von wenigstens 7 cm, insbesondere von wenigstens 8 cm, insbesondere von wenigstens 9 cm, insbesondere von wenigstens 10 cm, insbesondere von höchstens 20 cm, insbesondere von 10 -18 cm, insbesondere von 10 - 16 cm aufweist.

2. Fixier- und Tragevorrichtung nach Anspruch 1, **dadurch gekennzeichnet, dass** der Rückenbereich (58) der angelegten Inkontinenzvorlage (4) durch den hinteren Rückenbereich (16) des Hüftgürtels und/oder den hinteren Latzabschnitt (34) zu mindestens 60% überdeckt ist, insbesondere zu mindestens 70%.

3. Fixier- und Tragevorrichtung nach Anspruch 1 oder 2, **dadurch gekennzeichnet, dass** der Bauchbereich (60) der angelegten Inkontinenzvorlage (4) durch den vorderen Bauchbereich (14) des Hüftgürtels und/oder den vorderen Latzabschnitt (32) zu mindestens 50%, vorzugsweise mindestens 60% und vorzugsweise mindestens 70% überdeckt ist.

4. Fixier- und Tragevorrichtung nach einem oder mehreren der vorangehenden Ansprüche, **dadurch gekennzeichnet, dass** die Inkontinenzvorlage (4) zwei Längs- und zwei Querkanten umfasst und im an der Fixier- und Tragevorrichtung (2) festgelegten Zustand, die Querkanten der Inkontinenzvorlage (4) im wesentlichen mit einer Querkante, insbesondere der dem Schrittbereich abgewandten, also proximalen Querkante (70) des Hüftgürtels (6) zusammenfallen.

5. Fixier- und Tragevorrichtung nach einem oder mehreren der vorangehenden Ansprüche, **dadurch gekennzeichnet, dass** sich die Latzabschnitte (32, 34) in Richtung auf den Schrittbereich verjüngen, wobei die aufeinander zu laufenden Kanten der Latzabschnitte (32,34) gerade oder gekrümmt zur Ausbildung einer Konturierung sein können.

6. Fixier- und Tragevorrichtung nach einem oder mehreren der vorangehenden Ansprüche, **dadurch gekennzeichnet, dass** der Hüftgürtel (6) im hinteren Rückenbereich und/oder dem hiermit verbundenen Latzabschnitt (34) elastisch dehnbare Materialabschnitte (44) aufweist.

7. Fixier- und Tragevorrichtung nach Anspruch 6, **dadurch gekennzeichnet, dass** die elastisch dehnbaren Materialabschnitte (44) insbesondere an die Seitenbereiche (18, 20) anschließen und zwischen sich einen nicht elastisch dehnbaren Abschnitt (45) des Rückenbereichs (16) und/oder des hiermit verbundenen Latzabschnitts (34) einschließen, wobei die elastisch dehnbaren Materialabschnitte (44) insbesondere trapezförmig ausgebildet sind.

8. Fixier- und Tragevorrichtung nach Anspruch 6 oder 7, **dadurch gekennzeichnet, dass** die elastisch dehnbaren Materialabschnitte (44) so ausgebildet sind, dass ein absorbierender Kern (74), wenigstens hinsichtlich seiner Ränder (76), der mit dem Hüftgürtel (6) verbundenen Inkontinenzvorlage (4) im ungedehnten und im gedehnten Zustand der Materialabschnitte (44) im Bereich der Materialabschnitte (44) liegt.

9. Fixier- und Tragevorrichtung nach einem oder mehreren der vorangehenden Ansprüche, **dadurch gekennzeichnet, dass** die Verschlusselemente (40, 42) des Latzabschnitts (32, 34) auf einem streifenförmigen und quer zur Längsrichtung erstreckten Materialabschnitt vorgesehen sind, der an den Latzabschnitt (32, 34) angefügt ist.

10. Fixier- und Tragevorrichtung nach einem oder mehreren der vorangehenden Ansprüche, **dadurch gekennzeichnet, dass** die Verschlusselemente (40, 42) des Latzabschnitts (32, 34) vor der Ingebrauchnahme auf die körperzugewandte Seite des Latzabschnitts (32, 34) eingeschlagen sind und vorzugsweise dort haftend fixiert sind.

11. Fixier- und Tragevorrichtung nach einem oder mehreren der vorangehenden Ansprüche, **dadurch gekennzeichnet, dass** der Hüftgürtel (6) nur in einem Bereich öffen- und schließbar und die Gürtelverschlusselemente (22, 24) in diesem Bereich an freien Endabschnitten des Hüftgürtels (6) vorgesehen sind.

12. Fixier- und Tragevorrichtung nach einem oder mehreren der vorangehenden Ansprüche, **dadurch gekennzeichnet, dass** Sekundärverschlusselemente (26) vorgesehen sind, mittels derer eine Umfangslänge des Hüftgürtels (6) einstellbar und so zumindest der vordere Latzabschnitt (32, 34) symmetrisch zum Schritt des Benutzers positionierbar ist, wobei die Sekundärverschlusselemente (26) beidseits des Bauchbereichs (14) oder im Seitenbereich des Hüftgürtels (6), gegenüberliegend den Gürtelverschlusselementen (22) vorgesehen sind.

13. Fixier- und Tragevorrichtung nach einem oder mehreren der vorangehenden Ansprüche, **dadurch gekennzeichnet, dass** die Gürtelverschlusselemente (22) und/oder die Sekundärverschlusselemente (26) und/oder die Verschlusselemente (40,41) der Latzabschnitte (32,34) mechanische und/oder klebende Verschlusselemente sind.

14. System aus einer Fixier- und Tragevorrichtung (2) nach einem der vorangehenden Ansprüche mit einer daran lösbar festlegbaren Inkontinenzvorlage (4) mit einer Längsrichtung und einer Querrichtung sowie zwei Längskanten (54,56) und zwei Querkanten (50,52) sowieeinem Rückenbereich (58) und einem Bauchbereich (60) deren Abmessungen durch die Breite der Inkontinenzvorlage (4) quer zur Längsrichtung sowie die Erstreckung in Längsrichtung von einer Querkante (50,52) der Inkontinenzvorlage (4) bis zu einer distalen Kante (62,66) des die Inkontinenzvorlage (4) überdeckenden vorderen oder hinteren Latzabschnitts (32,34) bestimmt ist, **dadurch gekennzeichnet, dass** der Rückenbereich (58) der angelegten Inkontinenzvorlage (4) durch den hinteren Rückenbereich (16) des Hüftgürtels und/oder den hinteren Latzabschnitt (34) zu mindestens 50% überdeckt ist, insbesondere zu mindestens 60% und insbesondere zu mindestens 70%

15. System nach Anspruch 14, **dadurch gekennzeichnet, dass** der Bauchbereich (60) der angelegten Inkontinenzvorlage durch den vorderen Bauchbereich (14) des Hüftgürtels (6) und/oder den vorderen Latzabschnitt (32) zu mindestens 50% überdeckt sind, insbesondere zu mindestens 60% und insbesondere zu mindestens 70%.

16. System nach Anspruch 14 oder 15, **dadurch gekennzeichnet, dass** bei der Inkontinenzvorlage (4) im an der Fixier- und Tragevorrichtung festgelegten Zustand, die Querkanten (50,52) im Wesentlichen mit einer Querkante (70), insbesondere der dem Schrittbereich abgewandten, also proximalen Querkante (70) des Hüftgürtels (6) zusammenfallen.

17. System nach einem oder mehreren der vorangehenden Ansprüche, **dadurch gekennzeichnet, dass** der Hüftgürtel (6) im hinteren Rückenbereich und/oder dem hiermit verbundenen Latzabschnitt (34) elastisch dehnbare Materialabschnitte (44) aufweist, wobei die elastisch dehnbaren Materialabschnitte (44) insbesondere an die Seitenbereiche (18, 20) anschließen und zwischen sich einen nicht elastisch dehnbarenAbschnitt (45) des Rückenbereichs (16) und/oder des hiermit verbundenen Latzabschnitts (34) einschließen, wobei die elastisch dehnbaren Materialabschnitte (44) insbesondere trapezförmig ausgebildet sind und wobei die elastisch dehnbaren Materialabschnitte (44) so ausgebildet sind, dass der absorbierende Kern (74) der mit dem Hüftgürtel (6) verbundenen Inkontinenzvorlage (4) wenigstens hinsichtlich seiner Ränder (76) im ungedehnten und im gedehnten Zustand der Materialabschnitte (44) im Bereich der Materialabschnitte (44) liegt.

## Claims

1. Fixing and carrying device (2) for a disposable absorbent incontinence pad (4) having a hip belt (6) that can be releasably closed on itself by means of belt fastening elements (22) and thus forms a hip opening that is continuous in the circumferential direction (8), to which hip belt the incontinence pad (4) can be releasably attached so that it can be worn in the crotch region of the user and removed again from the hip belt (6) and discarded after use, the hip belt (6) having a front stomach region (14), a rear back region (16) and a left and a right side region (18, 20), the hip belt (6), starting from the back region (16) and the stomach region (14), having a flap portion (32, 34) which extends in a longitudinal direction in the direction (38) of the crotch region of the user and which has fastening elements (40, 42) on the side thereof facing the body, which fastening elements interact in a releasably adherent manner with complementary fastening elements on the side of the incontinence pad (4) facing away from the body, the incontinence pad (4) having a back region (58) and a stomach region (60), the dimensions of which are determined by the width of the incontinence pad (4) transversely to the longitudinal direction and the extension in the longitudinal direction from a transverse edge (50, 52) of the incontinence pad (4) up to a distal edge (62, 66) of the front or rear flap portion (32, 34) covering the incontinence pad (4), **characterized in that** at least 50% of the back region (58) of the applied incontinence pad (4) is covered by the rear back region (16) of the hip belt and the rear flap portion (34), and the flap portion has an extension in the longitudinal direction over a distal transverse edge of the stomach or back region facing the crotch in the distal direction toward the crotch region of at least 5 cm, in particular of at least 6 cm, in particular of at least 7 cm, in particular of at least 8 cm, in particular of at least 9 cm, in particular of at least 10 cm, in particular of at most 20 cm, in particular of 10-18 cm, in particular of 10-16 cm.

2. Fixing and carrying device according to claim 1, **characterized in that** at least 60%, in particular at least 70%, of the back region (58) of the applied incontinence pad (4) is covered by the rear back region (16) of the hip belt and/or the rear flap portion (34).

3. Fixing and carrying device according to either claim 1 or claim 2, **characterized in that** at least 50%, preferably at least 60% and preferably at least 70%, of the stomach region (60) of the applied incontinence pad (4) is covered by the front stomach region (14) of the hip belt and/or the front flap portion (32).

4. Fixing and carrying device according to one or more of the preceding claims, **characterized in that** the incontinence pad (4) comprises two longitudinal and two transverse edges and, in the state fixed on the fixing and carrying device (2), the transverse edges of the incontinence pad (4) substantially coincide with a transverse edge, in particular the transverse edge (70) of the hip belt (6) facing away from the crotch region, i.e. the proximal transverse edge.

5. Fixing and carrying device according to one or more of the preceding claims, **characterized in that** the flap portions (32, 34) taper in the direction of the crotch region, it being possible for the edges of the flap portions (32, 34) running towards one another to be straight or curved in order to form a contour.

6. Fixing and carrying device according to one or more of the preceding claims, **characterized in that** the hip belt (6) has elastically stretchable material portions (44) in the rear back region and/or the flap portion (34) connected thereto.

7. Fixing and carrying device according to claim 6, **characterized in that** the elastically stretchable material portions (44) in particular adjoin the side regions (18, 20) and include therebetween a non-elastically stretchable portion (45) of the back region (16) and/or the flap portion (34) connected thereto, the elastically stretchable material portions (44) being in particular trapezoidal.

8. Fixing and carrying device according to either claim 6 or claim 7, **characterized in that** the elastically stretchable material portions (44) are designed such that an absorbent core (74), at least with regard to its edges (76), of the incontinence pad (4) connected to the hip belt (6) is in the region of the material portions (44) in the unstretched state and in the stretched state of the material portions (44).

9. Fixing and carrying device according to one or more of the preceding claims, **characterized in that** the fastening elements (40, 42) of the flap portion (32, 34) are provided on a strip-shaped material portion extending transversely to the longitudinal direction, which material portion is attached to the flap portion (32, 34).

10. Fixing and carrying device according to one or more of the preceding claims, **characterized in that** the fastening elements (40, 42) of the flap portion (32, 34) are folded onto the side of the flap portion (32, 34) facing the body before being used and are preferably adherently fixed thereto.

11. Fixing and carrying device according to one or more of the preceding claims, **characterized in that** the hip belt (6) can only be opened and closed in one region and the belt fastening elements (22, 24) are provided in this region at free end portions of the hip belt (6).

12. Fixing and carrying device according to one or more of the preceding claims, **characterized in that** secondary fastening elements (26) are provided, by means of which a circumferential length of the hip belt (6) can be adjusted and thus at least the front flap portion (32, 34) can be positioned symmetrically to the crotch of the user, the secondary fastening elements (26) being provided on both sides of the stomach region (14) or in the side region of the hip belt (6) opposite the belt fastening elements (22) .

13. Fixing and carrying device according to one or more of the preceding claims, **characterized in that** the belt fastening elements (22) and/or the secondary fastening elements (26) and/or the fastening elements (40, 41) of the flap portions (32, 34) are mechanical and/or adhesive fastening elements.

14. System consisting of a fixing and carrying device (2) according to any of the preceding claims having an incontinence pad (4) which can be releasably fixed thereon with a longitudinal direction and a transverse direction as well as two longitudinal edges (54, 56) and two transverse edges (50, 52) and a back region (58) and a stomach region (60), the dimensions of which are determined by the width of the incontinence pad (4) transversely to the longitudinal direction and the extension in the longitudinal direction from a transverse edge (50, 52) of the incontinence pad (4) to a distal edge (62, 66) of the front or rear flap portion (32, 34) covering the incontinence pad (4), **characterized in that** at least 50%, in particular at least 60% and in particular at least 70%, of the back region (58) of the applied incontinence pad (4) is covered by the rear back region (16) of the hip belt and/or the rear flap portion (34).

15. System according to claim 14, **characterized in that** at least 50%, in particular at least 60% and in particular at least 70%, of the stomach region (60) of the applied incontinence pad is covered by the front stomach region (14) of the hip belt (6) and/or the front flap portion (32) .

16. System according to either claim 14 or claim 15, **characterized in that** in the state fixed on the fixing and carrying device of the incontinence pad the transverse edges (50, 52) substantially coincide with a transverse edge (70), in particular the transverse edge (70) of the hip belt (6) facing away from the crotch region, i.e. the proximal transverse edge.

17. System according to one or more of the preceding claims, **characterized in that** the hip belt (6) has elastically stretchable material portions (44) in the rear back region and/or the flap portion (34) connected thereto, the elastically stretchable material portions (44) in particular adjoining the side regions (18, 20) and including therebetween a non-elastically stretchable portion (45) of the back region (16) and/or the flap portion (34) connected thereto, the elastically stretchable material portions (44) being in particular trapezoidal, and the elastically stretchable material portions (44) being designed such that the absorbent core (74) of the incontinence pad (4) connected to the hip belt (6) is, at least with regard to its edges (76), in the region of the material portions (44) in the unstretched state and in the stretched state of the material portions (44) .

## Revendications

1. Dispositif de fixation et de support (2) pour une protection absorbante jetable pour l'incontinence (4), comprenant une ceinture de hanche (6) qui peut être fermée de manière amovible sur elle-même au moyen d'éléments de fermeture de ceinture (22) et forme ainsi une ouverture de hanche continue dans le sens du tour de hanches (8) et à laquelle la protection pour l'incontinence (4) peut être fixée de manière amovible de sorte qu'elle puisse être portée dans la zone d'entrejambe de l'utilisateur et puisse être retirée à nouveau de la ceinture de hanche (6) et être jetée après l'utilisation, dans lequel la ceinture de hanche (6) présente une zone ventrale avant (14), une zone dorsale arrière (16) et des zones latérales gauche et droite (18, 20), la ceinture de hanche (6) comprenant, à partir de la zone dorsale (16) et de la zone ventrale (14), respectivement une section de bavette (32, 34) qui s'étend dans une direction longitudinale en direction (38) de la zone d'entrejambe de l'utilisateur et qui présente des éléments de fermeture (40, 42) sur sa face montrant vers le corps, qui coopèrent par adhérence amovible avec des éléments de fermeture complémentaires sur la face de la protection pour l'incontinence (4), qui montre dans la direction opposée au corps, dans lequel la protection pour l'incontinence (4) présente une zone dorsale (58) et une zone ventrale (60) dont les dimensions sont déterminées par la largeur de la protection pour l'incontinence (4) transversalement à la direction longitudinale ainsi que par l'extension dans la direction longitudinale allant d'un bord transversal (50, 52) de la protection pour l'incontinence (4) jusqu'à un bord distal (62, 66) de la section de bavette avant ou arrière (32, 34) recouvrant la protection pour l'incontinence (4), **caractérisé par le fait que** la zone dorsale (58) de la protection pour l'incontinence (4) appliquée est recouverte pour au moins 50 % par la zone dorsale arrière (16) de la ceinture de hanche et la section de bavette arrière (34), et que la section de bavette présente une extension dans la direction longitudinale sur un bord transversal distal de la zone ventrale ou dorsale, tourné vers l'entrejambe, dans le sens distal vers la zone de l'entrejambe, de 5 cm au moins, en particulier de 6 cm au moins, en particulier de 7 cm au moins, en particulier de 8 cm au moins, en particulier de 9 cm au moins, en particulier de 10 cm au moins, en particulier de 20 cm tout au plus, en particulier entre 10 et 18 cm, en particulier entre 10 et 16 cm.

2. Dispositif de fixation et de support selon la revendication 1, **caractérisé par le fait que** la zone dorsale (58) de la protection pour l'incontinence (4) appliquée est recouverte pour au moins 60 %, en particulier pour au moins 70 %, par la zone dorsale arrière (16) de la ceinture de hanche et/ou par la section de bavette arrière (34).

3. Dispositif de fixation et de support selon la revendication 1 ou 2, **caractérisé par le fait que** la zone ventrale (60) de la protection pour l'incontinence (4) appliquée est recouverte pour au moins 50 %, de préférence pour au moins 60 % et de préférence pour au moins 70 % par la zone ventrale avant (14) de la ceinture de hanche et/ou la section de bavette avant (32).

4. Dispositif de fixation et de support selon l'une ou plusieurs des revendications précédentes, **caractérisé par le fait que** la protection pour l'incontinence (4) comprend deux bords longitudinaux et deux bords transversaux et que, lorsqu'elle est fixée sur le dispositif de fixation et de support (2), les bords transversaux de la protection pour l'incontinence (4) coïncident pour l'essentiel avec un bord transversal, en particulier avec le bord transversal (70) de la ceinture de hanche (6), qui montre dans la direction opposée à la zone d'entrejambe, donc le bord transversal proximal.

5. Dispositif de fixation et de support selon l'une ou plusieurs des revendications précédentes, **caractérisé par le fait que** les sections de bavette (32, 34) se rétrécissent en direction de la zone d'entrejambe, dans lequel les bords des sections de bavette (32, 34), qui s'étendent les uns vers les autres peuvent être droits ou incurvés pour former un contourage.

6. Dispositif de fixation et de support selon l'une ou plusieurs des revendications précédentes, **caractérisé par le fait que** la ceinture de hanche (6) présente des sections de matériau élastiquement extensibles (44) dans la zone dorsale arrière et/ou la section de bavette (34) qui est reliée à celle-ci.

7. Dispositif de fixation et de support selon la revendication 6, **caractérisé par le fait que** les sections de matériau élastiquement extensibles (44) sont contiguës en particulier aux zones latérales (18, 20) et comprennent entre elles une section (45) qui n'est pas élastiquement extensible de la zone dorsale (16) et/ou de la section de bavette (34) qui est reliée à celle-ci, dans lequel les sections de matériau élastiquement extensibles (44) sont conçues en particulier de façon trapézoïdale.

8. Dispositif de fixation et de support selon la revendication 6 ou 7, **caractérisé par le fait que** les sections de matériau élastiquement extensibles (44) sont réalisées de telle sorte qu'un noyau absorbant (74), au moins en ce qui concerne ses bords (76), de la protection pour l'incontinence (4) reliée à la ceinture de hanche (6) est situé dans la zone des sections de matériau (44) lorsque les sections de matériau (44) se trouvent à l'état inétendu et à l'état étendu.

9. Dispositif de fixation et de support selon l'une ou plusieurs des revendications précédentes, **caractérisé par le fait que** les éléments de fermeture (40, 42) de la section de bavette (32, 34) sont prévus sur une section de matériau en forme de bande qui s'étend transversalement à la direction longitudinale et qui est attachée à la section de bavette (32, 34).

10. Dispositif de fixation et de support selon l'une ou plusieurs des revendications précédentes, **caractérisé par le fait que**, avant leur utilisation, les éléments de fermeture (40, 42) de la section de bavette (32, 34) sont repliés sur la face montrant vers le corps de la section de bavette (32, 34) et y sont de préférence fixés par adhérence.

11. Dispositif de fixation et de support selon l'une ou plusieurs des revendications précédentes, **caractérisé par le fait que** la ceinture de hanche (6) ne peut être ouverte et fermée que dans une zone et que les éléments de fermeture de ceinture (22, 24) dans cette zone sont prévus à des sections d'extrémité libres de la ceinture de hanche (6).

12. Dispositif de fixation et de support selon l'une ou plusieurs des revendications précédentes, **caractérisé par le fait que** des éléments de fermeture secondaires (26) sont prévus qui permettent de régler une longueur circonférentielle de la ceinture de hanche (6) et, ainsi, de positionner symétriquement à l'entrejambe de l'utilisateur au moins la section de bavette avant (32, 34), dans lequel les éléments de fermeture secondaires (26) sont prévus des deux côtés de la zone ventrale (14) ou dans la zone latérale de la ceinture de hanche (6), en regard des éléments de fermeture de ceinture (22).

13. Dispositif de fixation et de support selon l'une ou plusieurs des revendications précédentes, **caractérisé par le fait que** les éléments de fermeture de ceinture (22) et/ou les éléments de fermeture secondaires (26) et/ou les éléments de fermeture (40,41) des sections de bavette (32,34) sont des éléments de fermeture mécaniques et/ou adhésifs.

14. Système se composant d'un dispositif de fixation et de support (2) selon l'une quelconque des revendications précédentes, comprenant une protection pour l'incontinence (4) apte à y être fixée de manière amovible et ayant une direction longitudinale et une direction transversale et deux bords longitudinaux (54, 56) et deux bords transversaux (50,52) ainsi qu'une zone dorsale (58) et une zone ventrale (60) dont les dimensions sont déterminées par la largeur de la protection pour l'incontinence (4) transversalement à la direction longitudinale ainsi que par l'extension dans la direction longitudinale allant d'un bord transversal (50, 52) de la protection pour l'incontinence (4) jusqu'à un bord distal (62, 66) de la section de bavette avant ou arrière (32, 34) recouvrant la protection pour l'incontinence (4), **caractérisé par le fait que** la zone dorsale (58) de la protection pour l'incontinence (4) appliquée est recouverte pour au moins 50 %, en particulier pour au moins 60 % et en particulier pour au moins 70 % par la zone dorsale arrière (16) de la ceinture de hanche et/ou par la section de bavette arrière (34).

15. Système selon la revendication 14, **caractérisé par le fait que** la zone ventrale (60) de la protection pour l'incontinence (4) appliquée est recouverte pour au moins 50 %, en particulier pour au moins 60 % et en particulier pour au moins 70 % par la zone ventrale avant (14) de la ceinture de hanche et/ou la section de bavette avant (32) .

16. Système selon la revendication 14 ou 15, **caractérisé par le fait que**, lorsque la protection pour l'incontinence (4) est fixée sur le dispositif de fixation et de support, les bords transversaux (50,52) coïncident pour l'essentiel avec un bord transversal (70), en particulier avec le bord transversal (70) de la ceinture de hanche (6), qui montre dans la direction opposée à la zone d'entrejambe, donc le bord transversal proximal.

17. Système selon l'une ou plusieurs des revendications précédentes, **caractérisé par le fait que** la ceinture de hanche (6) présente des sections de matériau élastiquement extensibles (44) dans la zone dorsale arrière et/ou dans la section de bavette (34) reliée à celle-ci, dans lequel lesdites sections de matériau élastiquement extensibles (44) sont contiguës en particulier aux zones latérales (18, 20) et comprennent entre elles une section (45) qui n'est pas élastiquement extensible de la zone dorsale (16) et/ou de la section de bavette (34) qui est reliée à celle-ci, dans lequel les sections de matériau élastiquement extensibles (44) sont conçues en particulier de façon trapézoïdale, et dans lequel lesdites sections de matériau élastiquement extensibles (44) sont réalisées de telle sorte que le noyau absorbant (74) de la protection pour l'incontinence (4) reliée à la ceinture de hanche (6) est situé, au moins en ce qui concerne ses bords (76), dans la zone des sections de matériau (44) lorsque les sections de matériau (44) se trouvent à l'état inétendu et à l'état étendu
